# EUROPEAN PATENT SPECIFICATION

(11) **EP 2 522 745 B1**
(45) Date of publication and mention of the grant of the patent: **03.12.2014**
(21) Application number: 12167373.5
(22) Date of filing: 09.05.2012
(51) Int. Cl.: C12Q 1/68

(54) **Probe for detecting poly A repeat number polymorphism of HGF gene and uses thereof**
Sonde zum Erkennen des POLY-A-Wiederholungspolymorphismus des HGF-Gens und Verwendungen davon
Sonde pour détecter le polymorphisme de nombre de répétitions poly A du gène HGF et utilisations associées

(30) Priority: 09.05.2011 JP 2011104503; 10.04.2012 JP 2012089507
(43) Date of publication of application: 14.11.2012
(73) Proprietor: ARKRAY, Inc., Kyoto-shi, Kyoto 601-8045 (JP)
(72) Inventor: Iguchi, Aki, Kyoto-shi Kyoto 602-0008 (JP); Hirai, Mitsuharu, Kyoto-shi Kyoto 602-0008 (JP)
(74) Representative: Jones, Elizabeth Louise

(56) References cited:
- EP-A1- 2 055 774
- US-B1- 6 664 064
- JIHONG MA ET AL: "Somatic mutation and functional polymorphism of a novel regulatory element in the HGF gene promoter causes its aberrant expression in human breast cancer", JOURNAL OF CLINICAL INVESTIGATION, vol. 119, no. 3, 2 March 2009 (2009-03-02) , pages 478-491, XP55036717, ISSN: 0021-9738, DOI: 10.1172/JCI36640

## Description

The present invention relates to a probe for detecting poly-A repeat number Polymorphism in a promoter region of a HGF (Hepatocyte Growth Factor) gene and uses thereof.

Breast cancer is a carcinoma which occurs in breast tissue. The cancer is one seen often all over the world, and about 10% of women in Western countries have opportunity chance to suffer from breast cancer within their lifetimes. About 20% of female patients with breast cancer die of this disease. Therefore, a huge amount of effort has been spent to obtain early detection methods and effective therapeutic methods for the carcinoma.

In recent years, it turned out that a risk of developing breast cancer relates to a mutation of a poly-A repeat number in a promoter region of the HGF gene. Specifically, it turned out that the risk of developing breast cancer differs between the case where the poly-A repeat number of the promoter region of the HGF gene is 30 of normal type and the case where the poly-A repeat number has a truncating mutation that is, for example, 25 or less (Jihong Ma, et al., 2009, Somatic mutation and functional polymorphism of a novel regulatory element in the HGF gene promoter causes its aberrant expression in human breast cancer., The Journal of Clinical Investigation Volume 119 Number 3., 478-491).

One example of a method for detecting poly-A repeat number mutation in the HGF gene includes a method for detection by a Direct Sequencing method and electrophoresis. However, for example, in order to determine the difference in the repeat number of A between in the case where the poly-A repeat number is 30 and the case where the number is not more than 25, it is difficult to obtain an accurate base sequence since the waveform is disturbed when consecutive sequences of the same base are measured by the Direct Sequencing method. Even if electrophoresis is carried out, it is difficult to distinguish the difference in several bases, for example, between 30 and 25 bases or less, and therefore it is difficult to accurately determine whether the gene is a normal type or an abnormal type.

On the other hand, as a method for detecting gene polymorphism in recent years, a detection method utilizing melting curve analysis (Tm analysis) is used. This is a method for determining whether the gene polymorphism to be detected exists or not, the method comprising: using a probe complementary to a target sequence having a gene polymorphism to be detected; forming a hybrid (double-strand DNA) between a target single-strand DNA of a sample and the probe; performing a heat treatment on the hybrid; detecting dissociation (melting) of the hybrid accompanying the temperature rise by measuring signals such as absorbance and fluorescence intensity; and determining a Tm value (°C) based on the results of the detection. The Tm value (°C) increases as a homology (or an identity) between the hybrid increases, and the Tm value (°C) decreases as the homology decreases. Thus, by preliminarily calculating the Tm value (°C) (evaluation criteria value) of a hybrid oftarget sequence including the polymorphism to be detected and its complementary probe and then comparing the Tm value (°C) of the single-strand target sequence in the sample and the probe, it is possible to determine that there is the polymorphism to be detected in the target DNA if the measured value is equal to the evaluation criteria value, that is, a perfect match, and it is possible to determine that the subject polymorphism does not exists in the target DNA if the measured value is lower than the evaluation criteria value, in other words, a mismatch.

National Japanese Patent Publication No. 2007-510154 discloses an E. coli 16S rRNA-specific capture probe and an E. coli 16S rRNA-specific capture probe as described below (SEQ ID NO: 1 and SEQ ID NO: 2). However, these probes have A up to only 14 bases. In addition, the portion in which A is repeated is always located at the end of the sequences of the probes. Therefore, not only an abnormal type in which poly-A repeat number is not less than 15 but also an abnormal type in which a mutation of the poly-A repeat number in a promoter region of the HGF gene is sandwiched by other bases are undetectable by these probes.
SEQ ID NO: 1
5'-GCCAGCGTTCAATCTGAGCCATGATCAAACTCTTCAAAAAAAAAAAAAA-3'
SEQ ID NO: 2
5'-AAAAAAAAAAAAAAGCTGCCTCCCGTAGGAGT-3'

US 6664064 B1 discloses a method in which the number of repeat sequences which are present in a sample is determined by means of melting temperature analysis.

EP 2055774 A1 describes a primer set for amplifying a region including sites SULT1A1*2 and SULT1A1*3 in the SULT1A1 gene and a method comprising use of fluorescently labeled probes.

An object of the present invention is to provide a probe for detecting a polymorphism which can accurately discriminate a mutation of poly-A repeat number in a promoter region in a HGF gene, a method for detecting a polymorphism of the HGF gene using the probe for detecting a polymorphism, and a kit for detecting a polymorphism of the HGF gene including the probe for detecting a polymorphism.

The present inventors designed a probe for detecting a polymorphism which can discriminate a mutation of poly-A repeat number in a promoter region in the HGF gene, and discovered that the mutation of poly-A repeat number can more accurately be detected by performing Tm analysis using the probe for detecting a polymorphism, thereby completing the present invention.

The present invention is as follows:
<1> A probe for detecting a polymorphism of the HGF gene, characterized in that the probe consists of a fluorescent-labeled oligonucleotide selected from the group consisting of:
   a fluorescent-labeled oligonucleotide having the base sequence shown in SEQ ID NO: 5 wherein the 3' end base of the base sequence is labeled with a fluorescent dye;
   a fluorescent-labeled oligonucleotide having the base sequence shown in SEQ ID NO: 15 wherein the 3' end base of the base sequence is labeled with a fluorescent dye; and
   a fluorescent-labeled oligonucleotide having the base sequence shown in SEQ ID NO: 16 wherein the 3' end base of the base sequence is labeled with a fluorescent dye.
<2> The probe for detecting a polymorphism according to <1>, characterized in that the fluorescent-labeled oligonucleotide emits fluorescence when the oligonucleotide does not hybridize with a target sequence and that the fluorescence intensity of the oligonucleotide is decreased or increased when the oligonucleotide hybridizes with the target sequence.
<3> The probe for detecting a polymorphism according to <2>, characterized in that the fluorescent-labeled oligonucleotide emits fluorescence when the oligonucleotide does not hybridize with a target sequence and that the fluorescence intensity of the oligonucleotide is decreased when the oligonucleotide hybridizes with the target sequence.
<4> The probe for detecting a polymorphism according to any one of <1> to <3>, characterized in that the probe is a probe for melting curve analysis.
<5> A method for detecting a polymorphism of the HGF gene, characterized in that the probe according to any one of <1> to <4> is used. Alternatively expressed, use of the probe of any one of <1> to <4> for detecting a polymorphism in the HGF gene. The method may comprise contacting the probe of any one of <1> to <4> with a sample containing nucleic acid which may contain the polymorphism of the HGF gene and detecting the presence or absence of the polymorphism.
<6> The method for detecting a polymorphism of the HGF gene according to <5>, the method characterized by comprising:
   (I) a step of combining the probe for detecting a polymorphism according to any one of <1> to <4> and a single-strand nucleic acid in a sample to obtain a hybrid between the fluorescent-labeled oligonucleotide and the single strand nucleic acid;
   (II) a step of changing the temperature of the sample including said hybrid to dissociate said hybrid, followed by measuring changes of fluorescence signal based on dissociation of said hybrid;
   (III) a step of evaluating Tm which is a dissociation temperature of said hybrid based on said change of the fluorescence signal;
   (IV) a step of detecting the presence (or absence) of polymorphism of the HGF gene based on said Tm.
<7> The method for detecting a polymorphism of the HGF gene according to <6>, the method characterized by comprising a step of amplifying a nucleic acid before obtaining the hybrid in (I) or simultaneously with obtaining the hybrid in (I).
<8> A kit for detecting a polymorphism of the HGF gene, the kit characterized by comprising the probe for detecting a polymorphism according to any one of <1> to <4>.
<9> The kit for detecting a polymorphism of the HGF gene according to <8>, the kit characterized by comprising a primer capable of amplifying as a template a region comprising a sequence that hybridizes with at least one fluorescent-labeled oligonucleotide selected from the group consisting of a fluorescent-labeled oligonucleotide having the base sequence shown in SEQ ID NO: 5 wherein the 3' end base of the base sequence is labeled with a fluorescent dye, a fluorescent-labeled oligonucleotide having the base sequence shown in SEQ ID NO: 15 wherein the 3' end base of the base sequence is labeled with a fluorescent dye and a fluorescent-labeled oligonucleotide having the base sequence shown in SEQ ID NO: 16 wherein the 3' end base of the base sequence is labeled with a fluorescent dye. Also provided is the use of said kit for detecting said polymorphism.

A more complete understanding of this application can be obtained when the following detailed description is considered in conjunction with the following drawings, in which:
FIG. 1 is a diagram showing the result of the Tm analysis by 3T-HGF-WT-F1 probe according to Example 1;
FIG. 2 is a diagram showing the result of the Tm analysis by 3T-HGF-WT-F3 probe according to Example 2;
FIG. 3 is a diagram showing the result of the Tm analysis by 3T-HGF-WT-R1 probe according to Example 3;
FIG. 4 is a diagram showing the result of the Tm analysis by 3T-HGF-WT-F2 probe according to Comparative Example; and
FIG. 5 is a diagram showing the result of the Tm analysis by 3T-HGF-WT-R1 probe according to Example 4.

In the present invention, the target polymorphism to be detected basically means a mutation of poly-A repeat number in a promoter region in the HGF gene. The details of the HGF gene in the present invention are registered as a partial sequence in the National Center for Biotechnology Information (NCBI) under Accession No. NT_007933.15 (Homo sapiens chromosome 7 genomic contig, GRCh37. p2 reference primary assembly). The term "nt" in the present invention means the number of the base counted in the direction of 3'-end from 5'-end of the base sequence shown in SEQ ID NO: 3 which is the partial sequence, that is, nt1 to nt249. The base sequence of the nt1 to nt249 corresponds to the partial sequence of nt1943861 to nt1943310 of the entire base sequence of 77412220 bp registered under Accession No. NT_007933.15.

The number of poly-A repeat in a promoter region in human HGF gene is 30(31) if the gene is normal type, on the other hand if the gene is abnormal type, the number is 0(1) to 29(30). The term "poly-A repeat number mutation" in the present invention means the case where the gene is such abnormal type. In case where the poly-A repeat number is shown as, for example, 30(31), "30" means the number of T (or A complementary to T) from nt102 to nt131 which is the poly-A repeat number of a promoter region in HGF gene shown in SEQ ID NO:3, and "31" means the number of T (or A complementary to T) from nt101 to nt131 including the number of T arraying in the 5'-end of the poly-A repeat in the promoter region in the base sequence shown in SEQ ID NO: 3.

In the present invention, for the individual sequence of single strand nucleic acid to be detected in a sample, a probe or primer for detecting a polymorphism, matters based on complementary relation between these sequences are, unless otherwise specified, applied to sequences and also to sequences complementary to the respective sequences. When the matters of the present invention are applied to the sequences complementary to the respective sequences, sequences recognized by the complementary sequence shall be read as a corresponding sequence complementary to the sequence according to the present invention in the entire specification. The application is carried out within the scope of common general technical knowledge for those skilled in the art.

In the present invention, the term "Tm value" means a temperature at which double strand nucleic acid melts (melting temperature: Tm) and is generally defined as temperature at which absorbance at 260 nm reaches 50% of a total increase of absorbance. For example, when a solution containing double strand DNA is heated, the absorbance at 260 nm increases. This is because heating releases the hydrogen bond between both strands in the double strand DNA to dissociate it into single strand DNA(melting of DNA). When all double strand DNAs dissociate to single strand DNAs, the absorbance shows about 1.5 times of absorbance (absorbance of only double strand DNA) at the start time of heating. This allows a judgment of whether melting has completed or not. Tm value is set based on this phenomenon.

The term "step" in the present invention means a independent step, as well as a step which is not clearly distinguishable from other step provided that an expected action is accomplished by the step. In the present invention, numerical range shown using "to" shows a range which includes the numerical values indicated before and behind "to" as the minimum and the maximum, respectively. In the present invention, a quantity of respective components in a composition means, unless otherwise specified, the total quantity of a plurality of the substance which exists in the composition in the case where a plurality of substance corresponding to the respective components exists in the composition.

The term "target sequence" in the present invention means a nucleic acid sequence which is hybridized with the probe to detect polymorphism (mutation of poly-A repeat number). Probes for detecting a polymorphism according to the present invention will now be described. The term "including" and the term "having" include the meanings of "comprising" and "composed of". The term "an identity of at least (not less than) 80%" may indicate an identity of at least 85%, an identity of at least 90%, an identity of at least 95%, an identity of at least 96%, an identity of at least 97%, an identity of at least 98%, an identity of at least 99% to the sequence (or a part of said sequence corresponding to the sequence of the probe) from the viewpoint of a detection sensitivity. The relative % identity may be calculated based on the portion(s) of the base sequence or complementary base sequence corresponding to the probe described herein and having the same number of nucleotides as the probe, ignoring deletions, i.e. the % identity calculation may be performed by comparison of corresponding bases irrespective of the number of Ts in the base sequence or number of As in the complementary base sequence of SEQ ID NO: 3 compared to those in the probe.

Hybridization can be carried out according to known methods or methods according thereto such as a method described in Molecular Cloning 3rd (J. Sambrook et al., Cold Spring Harbor Lab. Press, 2001).

Hybridization as referred to herein is performed under stringent conditions. The term "under stringent conditions" as used herein means a condition in which a specific hybrid is formed but a nonspecific hybrid is not formed. Examples of typical stringent conditions include conditions in which hybridization is carried out in about 25 mM to about 50 mM of potassium, and about 1.0 mM to about 5.0 mM of magnesium. A detailed example includes a condition in which hybridization is carried out in Tris-HCl (pH8.6), 25 mM of KCl, and 1.5 mM of MgCl₂. Other examples of stringent conditions are described in Molecular Cloning 3rd (J. Sambrook et al., Cold Spring Harbor Lab. Press, 2001). Those skilled in the art may easily select such the conditions by changing hybridization reaction, salt concentration in hybridization solution and the like.

### <Probes for detecting a polymorphism>

A probe for detecting a polymorphism of HGF gene of the present invention is characterized in that the probe consists of a fluorescent-labeled oligonucleotide selected from the group consisting of:
a fluorescent-labeled oligonucleotide having the base sequence shown in SEQ ID NO: 5 wherein the 3' end base of the base sequence is labeled with a fluorescent dye;
a fluorescent-labeled oligonucleotide having the base sequence shown in SEQ ID NO: 15 wherein the 3' end base of the base sequence is labeled with a fluorescent dye; and
a fluorescent-labeled oligonucleotide having the base sequence shown in SEQ ID NO: 16 wherein the 3' end base of the base sequence is labeled with a fluorescent dye.

However, also disclosed herein are the following fluorescent-labelled oligonucleotides:
(P1-1) a fluorescent-labeled oligonucleotide wherein base sequence thereof is complementary to the base sequence of 8 to 94 bases comprising the base sequence from nt95 to nt100 and nt132 of base sequence shown in SEQ ID NO: 3 and the base sequence comprising 1 to 31 consecutive Ts sandwiched by the 3'-end of the base sequence from nt95 to nt100 and the nt132, and has an identity of at least not less than 80% to the base sequence complementary to the base sequence shown in SEQ ID NO: 3 under the condition that the base corresponding to nt95 is cytosine and wherein the base corresponding to nt95 is labeled with fluorescent dye;
(P1-2) a fluorescent-labeled oligonucleotide wherein base sequence thereof is complementary to the base sequence of 8 to 94 bases comprising the base sequence from nt95 to nt100 and nt132 of base sequence shown in SEQ ID NO: 3 and the base sequence comprising 1 to 31 consecutive Ts sandwiched by the 3'-end of the base sequence from nt95 to nt100 and the nt132, and the oligonucleotide hybridize with the base sequence shown in SEQ ID NO: 3 under stringent conditions, and wherein the base corresponding to nt95 is labeled with fluorescent dye;
(P1'-1) a fluorescent-labeled oligonucleotide wherein base sequence thereof is complementary to the base sequence of 7 to 55 bases comprising the base sequence from nt95 to nt100 of base sequence shown in SEQ ID NO: 3 and the base sequence comprising 1 to 31 consecutive Ts sandwiched by the 3'-end of the base sequence from nt95 to nt100 and nt132, and has an identity of at least not less than 80% to the base sequence complementary to the base sequence shown in SEQ ID NO: 3 under the condition that the base corresponding to nt95 is cytosine and wherein the base corresponding to nt95 is labeled with fluorescent dye;
(P1'-2) a fluorescent-labeled oligonucleotide wherein base sequence thereof is complementary to the base sequence of 7 to 55 bases comprising the base sequence from nt95 to nt100 of base sequence shown in SEQ ID NO: 3 and the base sequence comprising 1 to 31 consecutive Ts sandwiched by the 3'-end of the base sequence from nt95 to nt100 and nt132, and the oligonucleotide hybridize with the base sequence shown in SEQ ID NO: 3 under stringent conditions, and wherein the base corresponding to nt95 is labeled with fluorescent dye;
(P2-1) a fluorescent-labeled oligonucleotide wherein base sequence thereof is complementary to the base sequence of 11 to 94 bases comprising the base sequence from nt92 to nt100 and nt132 of base sequence shown in SEQ ID NO: 3 and the base sequence comprising 1 to 31 consecutive Ts sandwiched by the 3'-end of the base sequence from nt92 to nt100 and the nt132, and has an identity of at least not less than 80% to the base sequence complementary to the base sequence shown in SEQ ID NO: 3 under the condition that the base corresponding to nt92 is cytosine and wherein the base corresponding to nt92 is labeled with fluorescent dye;
(P2-2) a fluorescent-labeled oligonucleotide wherein base sequence thereof is complementary to the base sequence of 11 to 94 bases comprising the base sequence from nt92 to nt100 and nt132 of base sequence shown in SEQ ID NO: 3 and the base sequence comprising 1 to 31 consecutive Ts sandwiched by the 3'-end of the base sequence from nt92 to nt100 and the nt132, and the oligonucleotide hybridize with the base sequence shown in SEQ ID NO: 3 under stringent conditions, and wherein the base corresponding to nt92 is labeled with fluorescent dye;
(P2'-1) a fluorescent-labeled oligonucleotide wherein base sequence thereof is complementary to the base sequence of 10 to 55 bases comprising the base sequence from nt92 to nt100 of base sequence shown in SEQ ID NO: 3 and the base sequence comprising 1 to 31 consecutive Ts sandwiched by the 3'-end of the base sequence from nt92 to nt100 and nt132, and has an identity of at least not less than 80% to the base sequence complementary to the base sequence shown in SEQ ID NO: 3 under the condition that the base corresponding to nt92 is cytosine and wherein the base corresponding to nt92 is labeled with fluorescent dye;
(P2'-2) a fluorescent-labeled oligonucleotide wherein base sequence thereof is complementary to the base sequence of 10 to 55 bases comprising the base sequence from nt92 to nt100 of base sequence shown in SEQ ID NO: 3 and the base sequence comprising 1 to 31 consecutive Ts sandwiched by the 3'-end of the base sequence from nt92 to nt100 and nt132, and the oligonucleotide hybridize with the base sequence shown in SEQ ID NO: 3 under stringent conditions, and wherein the base corresponding to nt92 is labeled with fluorescent dye;
(P3-1) a fluorescent-labeled oligonucleotide wherein base sequence thereof is the base sequence of 8 to 120 bases comprising nt100 and the base sequence from nt132 to nt134 of base sequence shown in SEQ ID NO: 3 and the base sequence comprising 4 to 31 consecutive Ts sandwiched by the nt100 and the 5'-end of the base sequence from nt132 to nt134, and has an identity of at least not less than 80% to the base sequence shown in SEQ ID NO: 3 under the condition that the base of nt134 is cytosine and wherein the base of nt134 is labeled with fluorescent dye;
(P3-2) a fluorescent-labeled oligonucleotide wherein base sequence thereof is the base sequence of 8 to 120 bases comprising nt100 and the base sequence from nt132 to nt134 of base sequence shown in SEQ ID NO: 3 and the base sequence comprising 4 to 31 consecutive Ts sandwiched by the nt100 and the 5'-end of the base sequence from nt132 to nt134, and the oligonucleotide hybridize with base sequence complementary to the base sequence shown in SEQ ID NO: 3 under stringent conditions, and wherein the base of nt134 is labeled with fluorescent dye;
(P3'-1) a fluorescent-labeled oligonucleotide wherein base sequence thereof is the base sequence of 8 to 56 bases comprising the base sequence from nt132 to nt134 of base sequence shown in SEQ ID NO: 3 and the base sequence comprising 5 to 31 consecutive Ts sandwiched by nt100 and the 5'-end of the base sequence from nt132 to nt134, and has an identity of at least not less than 80% to the base sequence shown in SEQ ID NO: 3 under the condition that the base of nt134 is cytosine and wherein the base of nt134 is labeled with fluorescent dye;
(P3'-2) a fluorescent-labeled oligonucleotide wherein base sequence thereof is the base sequence of 8 to 56 bases comprising the base sequence from nt132 to nt134 of base sequence shown in SEQ ID NO: 3 and the base sequence comprising 5 to 31 consecutive Ts sandwiched by nt100 and the 3'-end of the base sequence from nt132 to nt134, and the oligonucleotide hybridize with base sequence complementary to the base sequence shown in SEQ ID NO: 3 under stringent conditions, and wherein the base of nt134 is labeled with fluorescent dye.

The probes of the invention having the base sequence shown in SEQ ID NOs: a) 5, b) 15 and c) 16, are examples of oligonucleotides a) (P1-1) and (P1-2); b) (P2-1) and (P2-2) and c) (P3-1) and (P3-2), respectively.

The fluorescent-labeled oligonucleotide of the present invention may be a fluorescent-labeled oligonucleotide whose fluorescence intensity is decreased (quenched) or increased when the oligonucleotide is hybridized with the complementary strand compared with a fluorescence intensity when the oligonucleotide is not hybridized with the complementary strand. Among these, the fluorescent -labeled oligonucleotide may be a fluorescent-labeled oligonucleotide whose fluorescence intensity is decreased when the oligonucleotide is hybridized with the complementary strand compared with a fluorescence intensity when the oligonucleotide is not hybridized with the complementary strand.

Probes utilizing such a quenching phenomenon are generally called guanine quenching probe and also known as what are called Q Probe^{®}. Among these, an example of a fluorescent-labeled oligonucleotide which is designed so that its 3'-end or 5'-end is cytosine (C) and the luminescence decreases when C at the ends approaches guanine (G) is especially. Use of such a probe enables easy confirmation of hybridization and dissociation by signal change.

Besides the method for detection using Q Probe ^{®}, known detection styles may also be applied. Examples of such detection styles include Taq-man Probe method, Hybridization Probe method, Moleculer Beacon method, MGB Probe method and the like.

Examples of the fluorescent dye include, but not restricted thereto, a fluorescein, a phosphor, a rhodamine, a polymethine dye derivative and the like. Examples of commercial products of such fluorescent dyes include Pacific Blue, BODIPY FL, FluorePrime, Fluoredite, FAM, Cy3, Cy5, TAMRA and the like.

Conditions for detecting a fluorescent-labeled oligonucleotide are not particularly restricted and can be appropriately determined depending on a fluorescent dye to be used. For example, Pacific Blue can be detected at a detection wavelength of 445 mn to 480 nm, TAMRA can be detected at a detection wavelength of 585 nm to 700 nm, and BODIPY FL can be detected at a detection wavelength of 520 nm to 555 nm. The uses of such fluorescent dyes make it possible to check hybridization and dissociation easily by signal changes. Binding a fluorescent dye to an oligonucleotide can be carried out according to usual methods described in, for example, Unexamined Japanese Patent Application Kokai Publication No. 2002-119291 or the like. Examples of the difference between a Tm value in the case where a fluorescent-labeled oligonucleotide hybridizes with DNA having a base sequence complementary to the fluorescent-labeled oligonucleotide and a Tm value in the case where a fluorescent-labeled oligonucleotide hybridizes with DNA having a base sequence in which only one base is not complementary to the fluorescent-labeled oligonucleotide include not less than 2.0°C, not less then 3.0°C, not less than 5.0°C or not less than 7.0°C.

A phosphate group may be added to the 3'-end of the probe for detecting a polymorphism of the present invention. As described later, a target sequence can be prepared by gene amplification methods such as PCR (polymerase chain reaction). In this case, the probe for detecting a polymorphism of the present invention may be made to coexist in a reaction solution of a gene amplification reaction. In such a case, addition of a phosphate group to the 3'-end of the probe for detecting a polymorphism sufficiently prevents the probe itself to elongate by the gene amplification reaction. The similar effect can be obtained by addition of the above-described labeled substance to the 3'-end of the probe.

The phrase "the base corresponding to nt95" of the fluorescent-labeled oligonucleotides of the (P1-1) and the (P1'-1) means the complementary base "C (cytosine)" corresponding to the base "G (guanine)" which is nt95 in the base sequence shown in SEQ ID NO: 3.

Concrete examples of (P1-1) variations include the following fluorescent-labeled oligonucleotide. (P1'-1), unlike the following (P1-1), is in the state that a base sequence comprising other kind of bases is attached only to the 3'-end of a sequence which is poly-A repeat portion.
poly-A repeat number 30(31), 46 mer, SEQ ID NO: 4
5'-CAGTTTGTGAAAAAAAAAAAAAAAAAAAAAAAAAAAAAAAGCTGCC-(fluorescent-labeled)-3'
poly-A repeat number 30(31), 42 mer, SEQ ID NO: 5
5'-TTGTGAAAAAAAAAAAAAAAAAAAAAAAAAAAAAAAGCTGCC-(fluorescent-labeled)-3'
poly-A repeat number 0(1), 12 mer, SEQ ID NO: 6
5'-TTGTGAGCTGCC-(fluorescent-labeled)-3'.

The 3'-end fluorescent-labelled oligonucleotide having the base sequence shown in SEQ ID NO:5 is a probe according to the invention.

Next, "the base corresponding to nt92" of the fluorescent-labeled oligonucleotides of the (P2-1) and the (P2'-1) means the complementary base "C (cytosine)" corresponding to the base "G (guanine)" which is nt92 in the base sequence shown in SEQ ID NO: 3.

Concrete examples of (P2-1) variations include the following fluorescent-labeled oligonucleotide. (P2'-1), unlike the following (P2-1), is in the state that a base sequence comprising other kind of bases is attached only to the 3'-end of sequence which is poly-A repeat portion.
poly-A repeat number 30(31), 46 mer, SEQ ID NO: 7
5'-TTTGTGAAAAAAAAAAAAAAAAAAAAAAAAAAAAAAAGCTGCCTGC-(fluorescent-labeled)-3'
poly-A repeat number 30(31), 42 mer, SEQ ID NO: 8
5'-TGAAAAAAAAAAAAAAAAAAAAAAAAAAAAAAAGCTGCCTGC-(fluorescent-labeled )-3'
poly-A repeat number 0(1), 11 mer, SEQ ID NO: 9
5'-GAGCTGCCTGC-(fluorescent-labeled)-3'

The fluorescent-labelled oligonucleotide having the base sequence shown in SEQ ID NO:15 and labelled at the 3' end is a probe according to the invention which is an example of a (P2-1) oligonucleotide.

Finally, "the base of nt134" of the fluorescent-labeled oligonucleotides of the (P3-1) and the (P3'-1) means the base "C (cytosine)" which is nt134 in the base sequence shown in SEQ ID NO: 3.

Concrete examples of (P3-1) variations include the following fluorescent-labeled oligonucleotide. (P3'-1), unlike the following (P3-1), is in the state that a base sequence comprising other kind of bases is attached only to the 3'-end of sequence which is poly-A repeat portion.
poly-A repeat number 30(31), 46 mer, SEQ ID NO: 10
5'-AGAGCAGGCAGCTTTTTTTTTTTTTTTTTTTTTTTTTTTTTTTCAC-(fluorescent-labeled)-3'
poly-A repeat number 30(31), 36 mer, SEQ ID NO: 11
5'-GCTTTTTTTTTTTTTTTTTTTTTTTTTTTTTTTCAC-(fluorescent-labeled)-3'
poly-A repeat number 2(3), 8 mer, SEQ ID NO: 12
5'-GCTTTCAC-(fluorescent-labeled)-3'

The fluorescent-labelled oligonucleotide having the base sequence shown in SEQ ID NO:16 and labelled at the 3' end is a probe according to the invention which is an example of a (P3-1) oligonucleotide.

The probe for detecting a polymorphism according to the present invention can be used to detect a mutation of poly-A repeat number in HGF gene. The detection method is not restricted at all as long as it is a method utilizing hybridization of a target sequence with these probes for detecting a polymorphism. For example, it is possible to detect a mutation of poly-A repeat number in HGF gene using only the probes of the invention for detecting a polymorphism (normal type probe) of poly-A repeat number 30 (31). It is also possible to detect a mutation of poly-A repeat number in HGF gene using the probe of the invention for detecting various polymorphism (abnormal type probe) of except for poly-A repeat number 30 (31). The probe of the present invention is characterized in that it is a probe for melting curve analysis. The details of a method for detecting a polymorphism of HGF gene including such a method by melting curve analysis will now be described.

### <A method for detecting a polymorphism of HGF gene>

The method for detecting a polymorphism of HGF gene of the present invention (a method for detecting a mutation of poly-A repeat number in HGF gene) is characterized in that the above-mentioned probe for detecting a polymorphism is used. That is, as long as the method for detecting a polymorphism of the present invention is characterized in that the above-mentioned probe for detecting a polymorphism is used, other constitutions or conditions are not restricted to the following description.

The method for detecting a polymorphism of the present invention comprises, for example, the following step (I), step (II), step (III) and step (IV):
(I) a step of contacting the probe for detecting a polymorphism with a single strand nucleic acid in a sample to obtain a hybrid between the fluorescent-labeled oligonucleotide and the single strand nucleic acid;
(II) a step of changing temperature of a sample containing the hybrid and dissociating the hybrid to measure changes of fluorescence signal based on dissociation of the hybrid;
(III) a step of evaluating Tm value which is the melting temperature of the hybrid based on the changes of fluorescence signal;
(IV) a step of detecting the presence of polymorphism of HGF gene based on the Tm value.

In the step (III), evaluation of Tm value may only be evaluation (determination) of temperature at Tm value, or also contain evaluation (determination) of a height of a peak in Tm analysis, that is, a peak of the amount of the changes in fluorescence intensity. Abundance ratio of base sequences having a mutation of poly-A repeat number can be confirmed by evaluating the height of the peak.

More particularly, for example, when such a method for detecting a polymorphism is carried out by using only a normal type probe (a probe of the invention), Tm value in case of using a normal type single strand nucleic acid is measured in advance. Normal type single strand nucleic acid is isolated from genome DNA such as from whole blood using a commercially available kit for isolating DNA and used. Then, whether HGF gene in a sample has a mutation of poly-A repeat number or not can be evaluated by comparing the Tm value of the normal type and the Tm value of the sample. That is, when the Tm value is exactly the same value of a normal type, it is thought that poly-A repeat of the sample is in perfect match with that of normal type probe. However, when the Tm value is lower than normal type, it means that melting temperature of single strand nucleic acid in the sample and the normal type probe is low. In this case, it is seen that the poly-A repeat number of the single strand nucleic acid in the sample is lower than 30(31) of the normal type.

In both the case of not using the normal type probe or the case of using an abnormal type probe whose poly-A repeat number is 0 (1) to 29 (30), such the method can evaluate whether the Tm values are in perfect match or in mismatch and exactly detect a mutation of poly-A repeat number of sample. For such various probes, at least one kind of probe may be used, or two or more kind of probes may also be used together. Evaluation of a peak of the amount of the changes per unit time of fluorescence intensity (fluorescence signal) using at the same time two or more probes for detecting a polymorphism makes it possible to evaluate at once the abundance ratio of target sequences with poly-A repeat number which is in perfect match with the respective probes for detecting a polymorphism. Examples of concentrations of the probe for detecting a polymorphism of the present invention in the reaction system include, but not restricted thereto, 10 to 1000 nmol/L or 20 to 500 nmol/L per one kind of probe for detecting a polymorphism.

The method for detecting a polymorphism of the HGF gene according to the present invention may includes a step of amplifying a nucleic acid before obtaining the above-mentioned hybrid of (I) or simultaneously with obtaining the hybrid. Examples of methods for amplifying a nucleic acid include, but not restricted thereto, PCR method, NASBA (Nucleic Acid Sequence Based Amplification) method, TMA (Transcription-Mediated Amplification) method, SDA (Strand Displacement Amplification) or the like. A specific example of them is the PCR method.

The conditions of a method for amplifying a nucleic acid is not restricted, and can be carried out by a known method. A method for producing amplification product from a nucleic acid as a template includes a method using primers for amplifying a sequence including the mutation of poly-A repeat number to be detected. An amplification region of the primers is not restricted and can be appropriately set depending on the mutation of poly-A repeat number to be detected. Examples of the amplification regions include regions comprising sequences with which fluorescent-labeled oligonucleotides of the invention are hybridized. The sequences of the primers are not restricted as long as, for example, the sequence at which a mutation of poly-A repeat number occurs can be amplified, and can be appropriately set depending on the sequence to be detected and the surrounding sequence by known methods. Furthermore, the lengths of the primers are not restricted but can be set as general lengths, for example such as 10 to 50 bases.

Example of such primer includes, though either one of a forward primer (F primer) which amplifies a sense strand or a reverse primer (R primer) which amplifies a antisense strand can be used, use of a primer set in which both of them are used as a pair. Examples of concentrations of the primers in the reaction system using the primers include, but not restricted thereto, 0.1 to 4 µmol/L, 0.25 to 1.5 µmol/L or 0.5 to 1 µmol/L per one kind of primer. When F primer and R primer are used, examples of the ratio of F primer to R primer include, but not restricted thereto, 1 : 0.1 to 1 : 10, or 1 : 0.5 to 1 : 5 by mole (F primer : R primer).

Example of samples to which the method for detecting a polymorphism of HGF gene is applied includes, but is not restricted thereto, a biological sample. Specific examples of the biological samples include whole blood, hemocytes such as leukocyte cell, bone marrow, intraoral cells such as oral mucosa, somatic cells such as nail or hair, germ cells, expectoration, amniotic fluid, paraffin-embedded tissues, urine, gastric juice, stomach lavage fluid or the like. A method for obtaining the samples, a method for preparing a nucleic acid to be tested from the samples are not restricted and known methods can be applied thereto. When the biological sample consists of whole blood, examples of concentrations of the whole blood in the reaction system of the present invention include 0.01 to 2 v/v%, 0.05 to 1.5 v/v% or 0.1 to 1 v/v%. When the biological sample is a serum, examples of concentrations of the serum in the reaction system of the present invention include 0.1 to 20 v/v%, 0.25 to 15 v/v% or 0.5 to 10 v/v%.

### <Method for evaluating drugs>

The probes described herein may be used in a method for evaluating a drug in which the tolerance to the drug or an effect of the drug which can be used against diseases related to the HGF gene is evaluated by using the method for detecting a polymorphism of the HGF gene. That is, the method for evaluating a drug is characterized in using the method for polymorphism of the HGF gene, and other constitutions, conditions or the like are not restricted to the following description. The method is performed on a sample from a subject, preferably a human.

The method for evaluating a drug includes, for example, the following step (I) and step (II):
(I) a step of detecting a polymorphism of the HGF gene by the above-described method for detecting a polymorphism of the HGF gene;
(II) a step of evaluating tolerance to the drug or pharmacological effect of the drug based on the presence or absence of detected polymorphism.

Specific details are the same as the method for detecting a polymorphism of the HGF gene. Evaluation of tolerance to a drug or a pharmacological effect of a drug based on the presence or absence of detected polymorphism means carrying out evaluation of, for example, tolerance of breast cancer tissues or the like to a drug or a pharmacological effect of the drug based on the abundance ratio of mutation type and normal type or the presence or absence thereof. Such a method for evaluating a drug is useful to determine a therapeutic strategy for diseases which are caused by the mutation of poly-A repeat number, such as a change of drug dose, a change to another therapeutic drug or the like.

### <A kit for detecting a polymorphism of the HGF gene>

A kit for detecting a polymorphism of the HGF gene of the present invention is characterized in that the kit comprises the above-described probe of the invention for detecting a polymorphism. That is, the kit for detecting a polymorphism of the HGF gene of the present invention is characterized in that the kit comprises the above-described probe of the invention for detecting a polymorphism, and other constitutions, conditions or the like are not restricted to the following description. The kit may contain a primer for amplifying as a template a region comprising a base sequence with which the fluorescent-labeled oligonucleotide of the invention hybridizes.

An example of the kit for detecting a polymorphism of the HGF gene of the present invention will now be described. For example, the kit for detecting a polymorphism of the HGF gene of the present invention is a reagent kit for detecting a mutation of poly-A repeat number in HGF gene. In the reagent kit, the probe for detecting a polymorphism may be either one type of primer or two or more types of primers. In the latter case, two or more types of the primers may be contained in a mixed state or as separate reagents. When two or more types of the primers are contained in a mixed state in the kit for detecting a polymorphism of the HGF gene of the present invention or when the primers are contained as separate reagents and used together in a same reaction system of, for example, Tm dissociation analysis between each probe for detecting a polymorphism and each target sequence, each of the probes for detecting a polymorphism may be labeled with different fluorescent dyes. Changing the kind of the fluorescent dye as above, the probes for detecting a polymorphism can be detected respectively. Examples of the fluorescent dyes include substances with different detection wavelengths.

As described above, in recent years, it is said that a mutation of poly-A repeat number in the HGF gene relates to a risk of developing breast cancer. By using the probe for detecting a polymorphism, the method for detecting a polymorphism of the HGF gene and the kit for detecting a polymorphism of the present invention, an accurate detection of a poly-A repeat number mutation of several base units can be carried out, which enables a pre-exam of the risk for developing breast cancer, so the possibility of early detection of a breast cancer and the like is high.

Then examples of the present invention will now be described. However, the present invention is not restricted to the following examples.

Examples in which a mutation of poly-A repeat number in the region from 19432961th to 19432991th base which is a partial sequence of a promoter region in the HGF gene is evaluated using the probe for detecting a polymorphism of the present invention will now be described.

### (Example 1)

In Example 1, Tm analysis for a mutation of poly-A repeat number in a promoter region in the HGF gene was carried out using a full-automatic SNPs-testing devise (Trade Name: i-densy (IS-5310), produced by ARKRAY) and the probe for detecting a polymorphism of the present invention. Prescriptions of solutions and reagents of probes or the like to be added to i-densy (1S-5310) are shown in the following Table.

**[Table 1]**

| (Volume of reaction solution : 50 µl) | |
|---|---|
| 1 x PCR buffer | |
| dNTP | 0.2 mM |
| MgCl₂ | 1.5 mM |
| Taq polymerase | 1.88 U |
| Probe | 0.2 µM |
| Complementary strand | 0.2 µM |

The probe in the Table 1 is a 3T-HGF-WT-F1 probe (a probe for detecting normal type) and its sequence is listed below.
3T-HGF-WT-F1 probe, SEQ ID NO: 5,30(31)
5'-TTGTGAAAAAAAAAAAAAAAAAAAAAAAAAAAAAAAGCTGCC-(TAMRA)-3' TAMRA was used as fluorescent dye of 3T-HGF-WT-F1 probe for Tm analysis at C at the 3'-end of the sequence.

Two kinds of complementary strands which were hybridized with 3T-HGF-WT-F1 probe of the above sequence were used in this Example 1. An experiment using 0.1 µM each of the two kind of complementary strands was carried out. Sequences of each primer is listed below.
HGF-R-A30-60, SEQ ID NO: 13, 30(31)
5'-ctcagagcaggcagctttttttttttttttttttttttttttttttcacaaactgccaaa-3'
HGF-R-A25-60, SEQ ID NO: 14, 25(26)
5'-gggcteagageaggcagcttttttttttttttttttttttttttcacaaactgccaaact-3'

Such a reaction solution was added to i-densy (IS-5310) respectively, heated at 95°C for 1 second, then at 40°C for 60 seconds, and further heated from 40°C to 75°C at the rate of temperature rise of 1 °C per 3 seconds to measure changes of fluorescence intensity with time at the detection wavelength (585 to 700 nm) corresponding to fluorescent dyes of the probes, followed by carrying out Tm analysis.

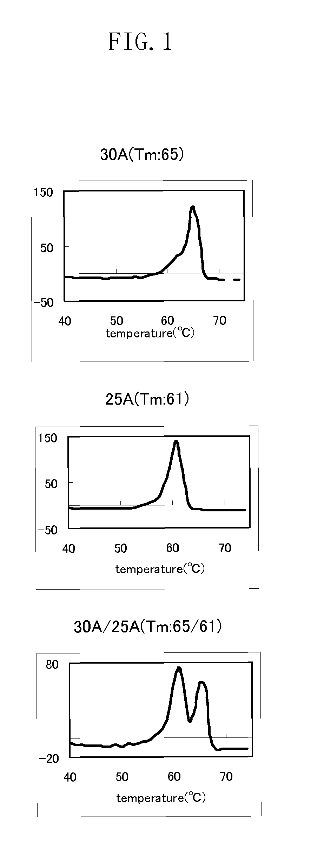

FIG. 1 shows a result of a Tm analysis with 3T-HGF-WT-F1 probe according to Example 1. The abscissa shows temperature (°C) and the ordinate shows the amount of the change of fluorescence intensity per unit time. In FIG.1, 30A shows a result of Tm analysis using 0.2 µM of complementary strand HGF-R-A30-60, 25A is a result using 0.2 µM of complementary strand HGF-R-A25-60, and 30A/25A is a result using 0.1 µM of each complementary strand. As shown with 30A in FIG.1, when poly-A repeat number was 30 (31) (in case of normal type), the peak was observed at 65°C, which showed the Tm value increased. In spite of the fact that poly-A repeat number of 25A was only several base less than that of normal type, its peak was observed at 61°C, which showed that the Tm value decreased to distinguish peak of 25A from that of 30A. Furthermore, it can be seen that the peak was observed at both Tm values in 30A/25A comprising 0.1 µM of each strand.

### (Example 2)

In Example 2, Tm analysis was carried out using a probe for detecting a polymorphism of the present invention which is different from the probe for detecting a polymorphism of the present invention used in Example 1. Experimental protocol and combination of reagents or the like was as in the above-described Example 1.

In this Example 2, 3T-HGF-WT-F3 probe was used in place of 3T-HGF-WT-F1 probe in Example 1. The sequence of 3T-HGF-WT-F3 probe is listed below.
3T-HGF-WT-F3 probe, SEQ ID NO: 15, 30(31)
5'-TTGTGAAAAAAAAAAAAAAAAAAAAAAAAAAAAAAAGCTGCCTGC-(TAMRA)-3'
Thus, TAMRA was used as fluorescent dye of 3T-HGF-WT-F3 probe for Tm analysis at C at the 3'-end of the sequence as in Example 1.

Two kinds of, and 0.1 µM each of the two kind of Complementary strands which hybridized with 3T-HGF-WT-F3 probe of the above sequence were used also in this Example 2 as in Example 1 as shown in the following sequence.
HGF-R-A30-60, SEQ ID NO: 13, 30(31)
5'-ctcagagcaggcagctttttttttttttttttttttttttttttttcacaaactgccaaa-3'
HGF-R-A25-60, SEQ ID NO: 14, 25(26)
5'-gggctcagagcaggcagcttttttttttttttttttttttttttcacaaactgccaaact-3'

Tm analysis was carried out on such a reaction solution by the same method as in Example 1.

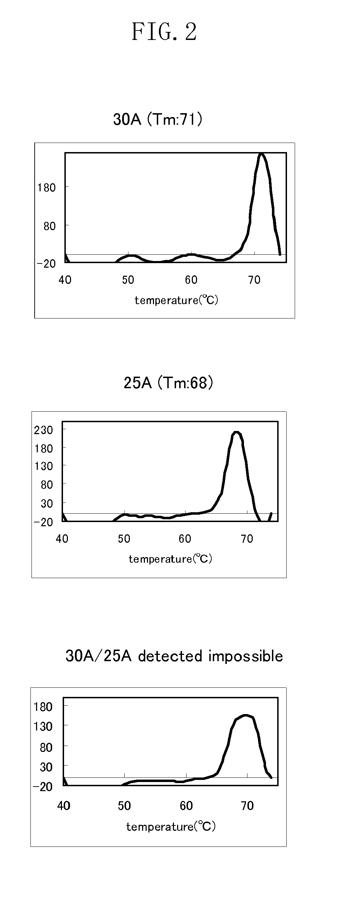

FIG. 2 shows a result of a Tm analysis with 3T-HGF-WT-F3 probe according to Example 2. The abscissa shows temperature (°C) and the ordinate shows the amount of the change of fluorescence intensity per unit of time. In FIG.2, 30A shows a result of Tm analysis using 0.2 µM of complementary strand HGF-F-A30-60, 25A is a result using 0.2 µM of complementary strand HGF-F-A25-60, and 30A/25A is a result using 0.1 µM of each complementary strand. As shown in 30A, when poly-A repeat number was 30 (31) (in case of normal type), the peak was observed at 71°C. In case with 25A, though there was no difference greater than the result of Example 1, the peak was observed at 68°C, which showed the Tm value decreased. However, in the case with 30A/25A comprising 0.1 µM of each strand, two clear peaks were not able to be detected presumably because there was not much difference between each peak of temperature.

### (Example 3)

Also in Example 3, Tm analysis was carried out using a probe for detecting a polymorphism of the present invention which is different from the probe used in Example 1 and Example 2. Experimental protocol and combination of reagents or the like was as in the above-described Example 1.

In this Example 3, 3T-HGF-WT-R1 probe was used in place of 3T-HGF-WT-F1 probe in Example 1. The sequence of 3T-HGF-WT-R1 probe is listed below.
3T-HGF-WT-R1 probe, SEQ ID NO: 16, 30(31)
5'-gctttttttttttttttttttttttttttttttcac-(TAMRA)-3'
Thus, TAMRA was used as fluorescent dye of 3T-HGF-WT-R1 probe for Tm analysis at C at the 3'-end of the sequence as in Example 1.

Two kinds of complementary strands which were hybridized with 3T-HGF-WT-R1 probe of the above sequence were used in the Example 3. An experiment using 0.1 µM each of the two kinds of complementary strands was carried out. Sequences of each strand is listed below.
HGF-F-A30-60, SEQ ID NO: 17, 30(31) HGF-F-A25-60, SEQ ID NO: 18, 25(26)

Tm analysis was carried out on such a reaction solution by the same method as in Example 1.

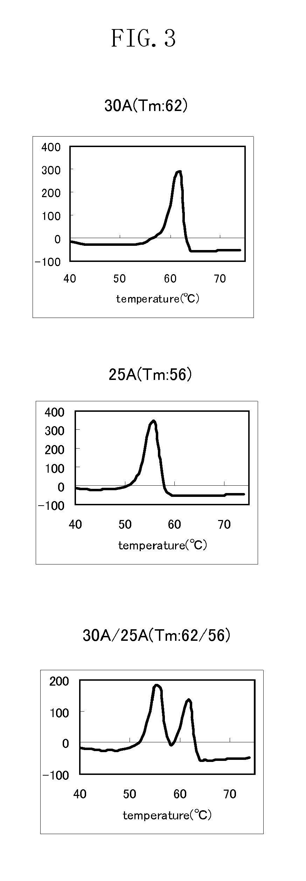

FIG. 3 shows a result of a Tm analysis with 3T-HGF-WT-R1 probe according to Example 3. The abscissa shows temperature (°C) and the ordinate shows the amount of the change of fluorescence intensity per unit of time. In FIG.3, 30A shows a result of Tm analysis using 0.2 µM of complementary strand HGF-F-A30-60, 25A is a result using 0.2 µM of complementary strand HGF-F-A25-60, and 30A/25A is a result using 0.1 µM of each complementary strand. First, as shown in 30A, when poly-A repeat number was 30 (31) (in case of normal type) and when 3T-HGF-WT-R1 was used, the peak was observed at 71°C. In 25A, similar to the result in Example 1, since the poly-A repeat number was decreased by several bases, its peak was observed at 56°C, which showed a great difference compared with Tm value of 30A.
It was possible to discriminate each peak in 30A/25A comprising 0.1 µM of each strand in the same way.

### (Comparative Example)

In Comparative Example, Tm analysis was carried out using probes which were different from the probe for detecting a polymorphism of the present invention. Experimental protocol and combination of reagents or the like was as in the above-described Example 1.

In this Comparative Example, 3T-HGF-WT-F2 probe was used in place of the probe in Example 1. The sequence of 3T-HGF-WT-F2 probe is listed below.
3T-HGF-WT-F2 probe, SEQ ID NO: 19, 30(31) Thus, TAMRA was used as fluorescent dye of 3T-HGF-WT-F2 probe for Tm analysis at C at the 3'-end of the sequence as in Example 1.

Two kinds of complementary strands which were hybridized with 3T-HGF-WT-F2 probe of the above sequence were used in this Comparative Example, which strands are similar to the Example 1 and Example 2 as shown in the sequence below.
HGF-R-A30-60, SEQ ID NO: 13, 30(31)
5'-ctcagagcaggcagctttttttttttttttttttttttttttttttcacaaactgccaaa-3'
HGF-R-A25-60, SEQ ID NO: 14, 25(26)
5'-gggctcagagcaggcagcttttttttttttttttttttttttttcacaaactgccaaact-3'

Tm analysis was carried out on such a reaction solution by the same method as in Example 1.

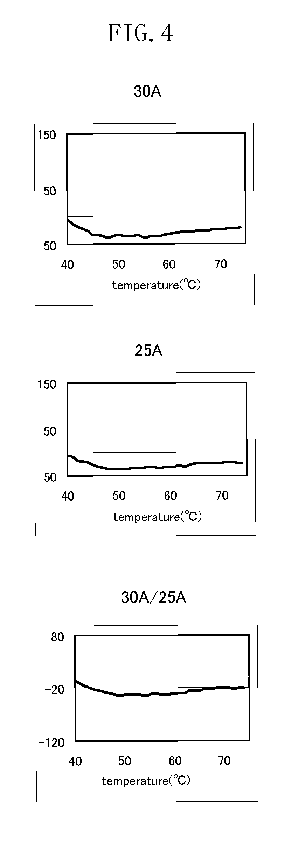

FIG. 4 shows a result of a Tm analysis with 3T-HGF-WT-F2 probe according to Comparative Example. The abscissa shows temperature (°C) and the ordinate shows the amount of the change of fluorescence intensity per unit time. In FIG.4, 30A shows a result of Tm analysis using 0.2 µM of complementary strand HGF-F-A30-60, 25A is a result using 0.2 µM of complementary strand HGF-F-A25-60, and 30A/25A is a result using 0.1 µM of each complementary strand. As shown in FIG.4, in any case of 30A, 25A and 30A/25A in this Comparative Example, the peak, that is, Tm value was not able to be detected.

From the results of Example 1 to Example 3, it was proven that a mutation of poly-A repeat number in a promoter region in HGF gene can be accurately confirmed by carrying out Tm analysis using a fluorescent label based on the position of "C" around the poly-A repeat sequence and evaluating a temperature of the peak value of signal of fluorescent label. However, from the results of Comparative Example, it was also confirmed that the position of "C" around the poly-A repeat sequence which is a basis for fluorescent-label was limited. That is, a mutation of poly-A repeat number in a promoter region in the HGF gene can be analyzed by fluorescent labeling of "C" which is a complementary base to G of nt95 in SEQ ID NO: 3 in Example 1, "C" which is a complementary base to G of nt92 in SEQ ID NO: 3 in Example 2, or "C" of nt134 in SEQ ID NO: 3 in Example 3.

### (Example 4)

In Example 4, Tm analysis during which PCR step was inserted was carried out for a mutation of poly-A repeat in HGF gene. As devises for carrying out PCR and TM analysis, i-densy (IS-5310) was used as in the above-described Example 1, Example 2 and Example 3. The composition of solution or reagent such as probe to be added was shown as in Table below.

**[Table 2]**

| (Volume of reaction solution : 50 µl) | |
|---|---|
| 1 x PCR buffer | |
| dNTP | 0.2 mM |
| MgCl₂ | 1.5 mM |
| Taq polymerase | 1.88 U |
| Probe | 0.2 µM |
| HGF-KKD-F | 1 µM |
| HGF-KKD-R | 0.5 µM |

HGF-KKD-F and HGF-KKD-R in Table 2 above are primers and their sequences are listed below.
HGF-KKD-F, SEQ ID NO: 20, 32 mer, Tm = 63.6°C
5'-GGGACAGGCTATGGACAATGACTGTTTCTTGG-3'
HGF-KKD-R, SEQ ID NO: 21, 28 mer, Tm = 61.4°C
5'-gggtgtggtattgtggggccaaaataag-3'

The probe in Table 2 above is 3T-HGF-WT-R1 probe as in the above-described Example 3 and its sequence is listed below.
3T-HGF-WT-R1 probe, SEQ ID NO: 16, 30(31)
5'-gctttttttttttttttttttttttttttttttcac-(TAMRA)-3'
TAMRA was used as fluorescent dye of 3T-HGF-WT-R1 probe for Tm analysis at C at the 3'-end of the sequence as mentioned above.

As a template, three kind of plasmid (all of which were produced by GeneScrip) were used and compared. The plasmid (29A) shown in SEQ ID NO: 22, the plasmid (25A) shown in SEQ ID NO: 23 and the plasmid (20A) shown in SEQ ID NO: 24 were used at 2500 copy/µl. Such a reaction solution was added to i-densy (IS-5310) respectively, and for carrying out PCR, heated at 95°C for 60 seconds, followed by performing 50 cycles of 95 °C for 1 second and 60 °C for 15 seconds. Then as in Example 1, Example 2 and Example 3 above, the solution was heated at 95°C for 1 second, then at 40°C for 60 seconds, and further heated from 40°C to 75°C at the rate of temperature rise of 1 °C per 3 seconds to measure changes of fluorescence intensity with time at the detection wavelength (5 85 to 700 nm) corresponding to fluorescent dyes of the probes, followed by carrying out Tm analysis.

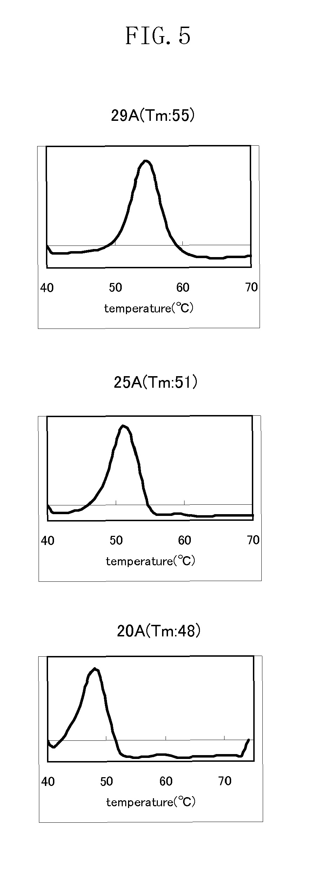

FIG. 5 shows a result of a Tm analysis with 3T-HGF-WT-R1 probe according to Comparative Example. The abscissa shows temperature (°C) and the ordinate shows the amount of the change of fluorescence intensity per unit time. FIG. 5 shows a result of Tm analysis using PCR in which 29A used a plasmid shown in SEQ ID NO: 22, 25A used a plasmid shown in SEQ ID NO: 23 and 20A used a plasmid shown in SEQ ID NO: 24. When poly-A repeat number is 29(30), the peak was observed at 55 °C when PCR was carried out as shown in 29A of FIG. 5. When poly-A repeat number is 25(26), the peak was observed at 51 °C when PCR was carried out as shown in 25A of FIG. 5. When poly-A repeat number is 20(21), the peak was observed at 48 °C when PCR was carried out as shown in 20A of FIG. 5, which showed that it is possible to discriminate each peak even if PCR step was included.

Whether accurate number of A base deletion can be determined or not was calculated using MeltCalc which is a software for calculating Tm value when changes of Tm value by A base deletion in poly-A repeat in target sequence at less unit than that in the above-described Example 1 to Example 3 were used as an index.

Firstly, normal type sequence and abnormal type sequence used in the calculation, that is, A base deleted sequences are listed below.
normal type, SEQ ID NO: 5, 30(31)
5'-TTGTGAAAAAAAAAAAAAAAAAAAAAAAAAAAAAAAGCTGCC-3'
poly-A repeat 1 base deletion, SEQ ID NO: 25, 29(30)
5'-AAAAAAAAAAAAAAAAAAAAAAAAAAAAAAGCTGCC-3'
poly-A repeat 3 base deletion, SEQ ID NO: 26, 27(28)
5'-AAAAAAAAAAAAAAAAAAAAAAAAAAAAGCTGCC-3'
poly-A repeat 5 base deletion, SEQ ID NO: 27, 25(26)
5'-AAAAAAAAAAAAAAAAAAAAAAAAAAGCTGCC-3'
normal type, SEQ ID NO: 15, 30(31)
5'-TTGTGAAAAAAAAAAAAAAAAAAAAAAAAAAAAAAAGCTGCCTGC-3'
poly-A repeat 1 base deletion, SEQ ID NO: 28, 29(30)
5'-gcaggcagcttttttttttttttttttttttttttttttt-3'
poly-A repeat 3 base deletion, SEQ ID NO: 29, 27(28)
5'-gcaggcagctttttttttttttttttttttttttttt-3'
poly-A repeat 5 base deletion, SEQ ID NO: 30, 25(26)
5'-gcaggcagctttttttttttttttttttttttttt-3'
normal type, SEQ ID NO: 16, 30(31)
5'-gctttttttttttttttttttttttttttttttcac-3'
poly-A repeat 1 base deletion, SEQ ID NO: 31, 29(30)
5'-ttttttttttttttttttttttttttttttcac-3'
poly-A repeat 3 base deletion, SEQ ID NO: 32, 27(28)
5'-ttttttttttttttttttttttttttttcac-3'
poly-A repeat 5 base deletion, SEQ ID NO: 33, 25(2b)
5'-ttttttttttttttttttttttttttcac-3'

The number of bases (mer), the number of A (t) and calculated Tm value (°C) in the normal sequence and the deleted sequence are listed below.

**[Table 3]**

| Sequence | mer | The number of A | Tm (°C) |
|---|---|---|---|
| Normal type 30A | 42 | 31 | 58.3 |
| 1-base deletion 29A | 36 | 30 | 55.2 |
| 3-bases deletion 27A | 34 | 28 | 54.6 |
| 5-bases deletion 25A | 32 | 26 | 53.9 |
| Normal type 30A | 45 | 31 | 60.6 |
| 1-base deletion 29A | 39 | 30 | 58.1 |
| 3-bases deletion 27A | 36 | 28 | 57.7 |
| 5-bases deletion 25A | 34 | 26 | 57 |
| Normal type 30A | 36 | 31 | 53.3 |
| 1-base deletion 29A | 33 | 30 | 50.3 |
| 3-bases deletion 27A | 31 | 28 | 49.3 |
| 5-bases deletion 25A | 29 | 26 | 48.2 |

In order to study in detail base length and base sequence of the fluorescent-labeled probe by which the same effect can be obtained, the relation between Tm value (°C) and its change and base sequence was examined by the same method as in Example 1 to Example 3.

First, base sequences of probes which was studied by fluorescent-labeling of a base corresponding to nt95 of SEQ ID NO:3 are listed below.
SEQ ID NO: 34
5'-AGCTGCC-3'
SEQ ID NO: 35
5'-GAGCTGCC-3'
SEQ ID NO: 36
5'-TTGTGAGCTGCC-3'
SEQ ID NO: 37
5'-AAAGCTGCC-3'
SEQ ID NO: 38
5'-AAAAAAGCTGCC-3'
SEQ ID NO: 39
5'-AAAAAAAAAAAAAAAAAAAAAAAAAAAAAAAGCTGCC-3'
SEQ ID NO: 40
5'-TTGTGAAAAAAAAAAAAAAAAAAAAAAAAAAAAAAAGCTGCC-3'
SEQ ID NO: 41
5'-AAAAAAAAAAAAAAAAAAAAAAAAAAAAAAAGCTGCCTGCTCTG-3'
SEQ ID NO: 42
5'-CAGTTTGTGAAAAAAAAAAAAAAAAAAAAAAAAAAAAAAAGCTGCC-3'
SEQ ID NO: 43
5'-AAAAAAAAAAAAAAAAAAAAAAAAAAAAAAAGCTGCCTGCTCTGAGCCCA-3'
SEQ ID NO: 44
5'-AAAAAAAAAAAAAAAAAAAAAAAAAAAAAAAGCTGCCTGCTCTGAGCCCATG-3'
SEQ ID NO: 45 SEQ ID NO: 46 SEQ ID NO: 47 SEQ ID NO: 48 SEQ ID NO: 49

Then, mer, the number of A, Tm value (°C) (perfect match), Tm value (°C) of 1 base deletion, the value (Δ) of (Tm value (°C) (perfect match) - Tm value (°C) of 1 base deletion) are listed below.

**[Table 4]**

| Base sequence of probe | mer | The number of A | Tm (PM) | Tm (1-mer deletion) | Δ |
|---|---|---|---|---|---|
| SEQ ID NO: 34 | 7 | 1 | 13.3 | - | |
| SEQ ID NO: 35 | 8 | 1 | 21.9 | - | |
| SEQ ID NO: 36 | 12 | 1 | 40.1 | - | |
| SEQ ID NO: 37 | 9 | 3 | 24.5 | - | |
| SEQ ID NO: 38 | 12 | 6 | 34.5 | - | |
| SEQ ID NO: 39 | 37 | 31 | 55.5 | 53.5 | 2.0 |
| SEQ ID NO: 40 | 42 | 31 | 58.3 | 55.2 | 3.1 |
| SEQ ID NO: 41 | 44 | 31 | 60.2 | 58.8 | 1.4 |
| SEQ ID NO: 42 | 46 | 31 | 60.0 | 55.2 | 4.8 |
| SEQ ID NO: 43 | 50 | 31 | 64.4 | 63.2 | 1.2 |
| SEQ ID NO: 44 | 52 | 31 | 64.6 | 63.5 | 1.1 |
| SEQ ID NO: 45 | 55 | 31 | 66.7 | 65.7 | 1.0 |
| SEQ ID NO: 46 | 56 | 31 | 67.6 | 66.7 | 0.9 |
| SEQ ID NO: 47 | 57 | 31 | 68.0 | 67.1 | 0.9 |
| SEQ ID NO: 48 | 94 | 31 | 73.1 | 72.1 | 1.0 |
| SEQ ID NO: 49 | 99 | 31 | 73 | 72.1 | 0.9 |

Provided that the probe for detecting a polymorphism of the present invention could detect polymorphism only when the value (Δ) was not less than 1°C (including undetectable) and the Tm value of the probe itself was not less than 0(°C), it was suggested that the fluorescent-labeled probes of SEQ ID NO: 46, SEQ ID NO: 47 and SEQ ID NO: 49 in Table 4 above could not detect polymorphism.

Furthermore, base sequences of probes which was studied by fluorescent-labeling of a base corresponding to nt92 of SEQ ID NO:3 are listed below.
SEQ ID NO: 50
5'-AGCTGCCTGC-3'
SEQ ID NO: 51
5'-GAGCTGCCTGC-3'
SEQ ID NO: 52
5'-AAAGCTGCCTGC-3'
SEQ ID NO: 53
5'-AAAAAAGCTGCCTGC-3'
SEQ ID NO: 54
5'-AAAAAAAAAAAAAAAAAAAAAAAAAAAAAAAGCTGCC-3'
SEQ ID NO: 55
5'-AAAAAAAAAAAAAAAAAAAAAAAAAAAAAAAGCTGCCTGC-3'
SEQ ID NO: 56
5'-TGAAAAAAAAAAAAAAAAAAAAAAAAAAAAAAAGCTGCCTGC-3'
SEQ ID NO: 57
5'-AAAAAAAAAAAAAAAAAAAAAAAAAAAAAAAGCTGCCTGCTC-3'
SEQ ID NO: 58
5'-TTTGTGAAAAAAAAAAAAAAAAAAAAAAAAAAAAAAAGCTGCCTGC-3'
SEQ ID NO: 59 SEQ ID NO: 60 SEQ ID NO: 61 SEQ ID NO: 62

Then, mer, the number of A, Tm value (°C) (perfect match), Tm value (°C) of 1 base deletion, the value (Δ) of (Tm value (°C) (perfect match) - Tm value (°C) of 1 base deletion) are listed below.

**[Table 5]**

| Base sequence of probe | mer | The number of A | Tm(PM) | Tm (1-merdeletion) | Δ |
|---|---|---|---|---|---|
| SEQ ID NO: 50 | 10 | 1 | 36.5 | 29 | 7.5 |
| SEQ ID NO: 51 | 11 | 1 | 40.3 | 38.9 | 1.4 |
| SEQ ID NO: 52 | 12 | 3 | 41.2 | 32.8 | 8.4 |
| SEQ ID NO: 53 | 15 | 6 | 46 | 39.9 | 6.1 |
| SEQ ID NO: 54 | 37 | 31 | 55.5 | 53.5 | 2.0 |
| SEQ ID NO: 55 | 40 | 31 | 58.3 | 56.6 | 1.7 |
| SEQ ID NO: 56 | 42 | 31 | 59.4 | 54.4 | 5 |
| SEQ ID NO: 57 | 42 | 31 | 59.2 | 57.7 | 1.5 |
| SEQ ID NO: 58 | 46 | 31 | 60.7 | 52.7 | 8 |
| SEQ ID NO: 59 | 55 | 31 | 66.7 | 65.7 | 1.0 |
| SEQ ID NO: 60 | 57 | 31 | 68 | 67.1 | 0.9 |
| SEQ ID NO: 61 | 94 | 31 | 73.1 | 72.1 | 1.0 |
| SEQ ID NO: 62 | 99 | 31 | 73 | 72.1 | 0.9 |

Provided that the probe for detecting a polymorphism of the present invention can detect polymorphism under the same conditions as described above, it was suggested that the fluorescent-labeled probes of SEQ ID NO: 60 and SEQ ID NO: 62 in Table 5 above could not detect polymorphism.

Furthermore, base sequences of probes which was studied by fluorescent-labeling of a base of nt134 of SEQ ID NO:3 are listed below.
SEQ ID NO: 63
5'-CTCAC-3'
SEQ ID NO: 64
5'-CTTCAC-3'
SEQ ID NO: 65
5'-TTTCAC-3'
SEQ ID NO: 66
5'-CTTTCAC-3'
SEQ ID NO: 67
5'-TTTTCAC-3'
SEQ ID NO: 68
5'-CTTTTCAC-3'
SEQ ID NO: 69
5'-TTTTTCAC-3'
SEQ ID NO: 70
5'-TTTTTTTCAC-3'
SEQ ID NO: 71
5'-TTTTTTTTTTTTTTTTTTTTTTTTTTTTTTTCAC-3'
SEQ ID NO: 72
5'-CTTTTTTTTTTTTTTTTTTTTTTTTTTTTTTTCAC-3'
SEQ ID NO: 73
5'-GCTTTTTTTTTTTTTTTTTTTTTTTTTTTTTTTCAC-3'
SEQ ID NO: 74
5'-AGAGCAGGCAGCTTTTTTTTTTTTTTTTTTTTTTTTTTTTTTTCAC-3'
SEQ ID NO: 75 SEQ ID NO: 76 SEQ ID NO: 77 SEQ ID NO: 79

Then, mer, the number of A, Tm value (°C) (perfect match), Tm value (°C) of 1 base deletion, the value (Δ) of (Tm value (°C) (perfect match) - Tm value (°C) of 1 base deletion) are listed below.

**Table 6**

| Base sequence of probe | mer | The number of A | Tm (PM) | Tm (1-mer deletion) | Δ |
|---|---|---|---|---|---|
| SEQ ID NO: 63 | 5 | 1 | -24.9 | - | - |
| SEQ ID NO: 64 | 6 | 2 | -11.2 | - | - |
| SEQ ID NO: 65 | 6 | 3 | -5.3 | - | |
| SEQ ID NO: 66 | 7 | 3 | -1.2 | - | - |
| SEQ ID NO: 67 | 7 | 4 | -4.3 | - | |
| SEQ ID NO: 68 | 8 | 4 | 6.6 | - | |
| SEQ ID NO: 69 | 8 | 5 | 4 | - | |
| SEQ ID NO: 70 | 10 | 7 | 15.9 | 7 | 8.9 |
| SEQ ID NO: 71 | 34 | 31 | 50.8 | 48.9 | 1.9 |
| SEQ ID NO: 72 | 35 | 31 | 51.5 | 46.7 | 4.8 |
| SEQ ID NO: 73 | 36 | 31 | 53.3 | 50.3 | 3.0 |
| SEQ ID NO: 74 | 46 | 31 | 61.2 | 59.6 | 1.6 |
| SEQ ID NO: 75 | 120 | 31 | 68.9 | 67.7 | 1.2 |
| SEQ ID NO: 76 | 145 | 31 | 70.7 | 69.8 | 0.9 |
| SEQ ID NO: 77 | 56 | 31 | 61.9 | 60.9 | 1 |
| SEQ ID NO: 78 | 60 | 31 | 63.6 | 62.7 | 0.9 |
| SEQ ID NO: 79 | 80 | 31 | 66.5 | 65.8 | 0.7 |

Provided that the probe for detecting a polymorphism of the present invention can detect polymorphism under the same conditions as described above, it was suggested that the fluorescent-labeled probes of SEQ ID NO: 63 to NO: 69, NO: 76, NO: 78 and NO: 79 in Table 6 above could not detect polymorphism.

More particularly, as shown in Table 4 to Table 6, depending on the position of labeling with fluorescent dye, there are cases where the number of consecutive Ts (or complementary A) and base length, the polymorphism may be detected or may not be detected.

For example, when the base corresponding to nt95 of SEQ ID NO: 3 was labeled with fluorescent dye, it was suggested that in a sequence having an identity of at least 80% to a base sequence complementary to a base sequence in which Ts were sandwiched between nt95 to nt100 and nt132, the number of consecutive Ts in the original base sequence were 1 to 31 and its base length is 8 to 94 (P1-1), on the other hand in a sequence having an identity of at least 80% to a base sequence complementary to a base sequence comprising the base sequence of nt95 to not100 and consecutive Ts (between which consecutive Ts were not sandwiched), the number of consecutive Ts in the original base sequence were 1 to 31 and its base length is 7 to 55 {P1'-1}.

When the base corresponding to nt92 of SEQ ID NO: 3 was labeled with fluorescent dye, it was suggested that in a sequence having an identity of at least 80% to a base sequence complementary to a base sequence in which Ts were sandwiched between nt92 to nt100 and nt132, the number of consecutive Ts in the original base sequence were 1 to 31 and its base length is 11 to 94 (P2-1), on the other hand in a sequence having an identity of at least 80% to a base sequence complementary to a base sequence comprising the base sequence of nt92 to nt100 and consecutive Ts (between which consecutive Ts were not sandwiched), the number of consecutive Ts in the original base sequence were 1 to 31 and its base length is ID to 55 (P2'-1). When the base of nt134 of SEQ ID NO: 3 was labeled with fluorescent dye, it was suggested that in a sequence having an identity of at least 80% to a base sequence in which Ts were sandwiched between nt100 and nt132 to nt134, the number of consecutive Ts in the original base sequence were 4 to 31 and its base length is 8 to 120 (P3-1), on the other hand in a sequence having an identity of at least 80% to a base sequence comprising the base sequence of nt132 to nt134 and consecutive Ts (between which consecutive Ts were not sandwiched), the number of consecutive Ts in the original base sequence were 5 to 31 and its base length is 8 to 56 (P3'-1). The invention extends to a probe which consists of a 3'-fluorescently-labelled oligonucleotide having the base sequence set forth in SEQ ID NO:5, 15 or 16.

Thus, by utilizing the present invention, a mutation of a poly-A repeat number in a promoter region in HGF gene, even several base units, can be accurately detected, and the poly-A repeat number, even several base units, can be accurately determined even when normal type and abnormal type co-exist. Especially, when Tm analysis is simultaneously utilized, evaluation of melting curve enables easy determination of a mutation of poly-A repeat number, no matter whether one kind or more than two kinds. Further, as described in Example and Comparative Example, since the position to be labeled with fluorescent dye which enables evaluation of a mutation of a poly-A repeat number is restricted, it can be said that the probe for detecting a polymorphism and the method for detecting a polymorphism in the HGF gene which utilize the position to be labeled according to the present invention.

Having described and illustrated the principles of this application by reference to one (or more) preferred embodiment(s), it should be apparent that the preferred embodiment(s) may be modified in arrangement and detail without departing from the principles disclosed herein and that it is intended that the application be construed as including all such modifications and variations insofar as they come within the spirit and scope of the subject matter disclosed herein and fall within the scope of the appended claims.

## Claims

1. A probe for detecting a polymorphism of the HGF gene, **characterized in that** the probe consists of a fluorescent-labeled oligonucleotide selected from the group consisting of:
a fluorescent-labeled oligonucleotide having the base sequence shown in SEQ ID NO: 5 wherein the 3' end base of the base sequence is labeled with a fluorescent dye;
a fluorescent-labeled oligonucleotide having the base sequence shown in SEQ ID NO: 15 wherein the 3' end base of the base sequence is labeled with a fluorescent dye; and
a fluorescent-labeled oligonucleotide having the base sequence shown in SEQ ID NO: 16 wherein the 3' end base of the base sequence is labeled with a fluorescent dye.

2. The probe for detecting a polymorphism according to claim 1, **characterized in that** the fluorescent-labeled oligonucleotide emits fluorescence when the oligonucleotide does not hybridize with a target sequence and that the fluorescence intensity of the oligonucleotide decreases or increases when the oligonucleotide hybridizes with the target sequence.

3. The probe for detecting a polymorphism according to claim 2, **characterized in that** the fluorescent-labeled oligonucleotide emits fluorescence when the oligonucleotide does not hybridize with a target sequence and that the fluorescence intensity of the oligonucleotide decreases when the oligonucleotide hybridizes with the target sequence.

4. The probe for detecting a polymorphism according to any one of claims 1 to 3, **characterized in that** the probe is a probe for melting curve analysis.

5. A method for detecting a polymorphism of the HGF gene, **characterized in that** the probe according to any one of claims 1 to 4 is used.

6. The method for detecting a polymorphism of the HGF gene according to claim 5, the method **characterized by** comprising:
(I) a step of combining the probe for detecting a polymorphism according to any one of claims 1 to 4 with a single-strand nucleic acid in a sample to obtain a hybrid between the fluorescent-labeled oligonucleotide and the single strand nucleic acid;
(II) a step of changing the temperature of the sample including the hybrid to dissociate the hybrid, followed by measuring changes in the fluorescence signal due to the dissociation of the hybrid;
(III) a step of evaluating Tm which is the dissociation temperature of the hybrid based on the change in the fluorescence signal;
(IV) a step of detecting the presence of the polymorphism of the HGF gene based on the Tm.

7. The method for detecting a polymorphism of the HGF gene according to claim 6, the method **characterized by** comprising a step of amplifying the nucleic acid before obtaining the hybrid in (I) or simultaneously with obtaining the hybrid in (I).

8. A kit for detecting a polymorphism of the HGF gene, the kit **characterized by** comprising the probe for detecting a polymorphism according to any one of claims 1 to 4.

9. The kit for detecting a polymorphism of the HGF gene according to claim 8, the kit **characterized by** comprising a primer capable of amplifying as a template a region comprising a sequence that hybridizes with at least one fluorescent-labeled oligonucleotide selected from the group consisting of a fluorescent-labeled oligonucleotide having the base sequence shown in SEQ ID NO: 5 wherein the 3' end base of the base sequence is labeled with a fluorescent dye, a fluorescent-labeled oligonucleotide having the base sequence shown in SEQ ID NO: 15 wherein the 3' end base of the base sequence is labeled with a fluorescent dye and a fluorescent-labeled oligonucleotide having the base sequence shown in SEQ ID NO: 16 wherein the 3' end base of the base sequence is labeled with a fluorescent dye.

## Patentansprüche

1. Eine Sonde zum Erkennen eines Polymorphismus von dem HGF Gen, **dadurch gekennzeichnet, dass** die Sonde aus einem fluoreszenzmarkierten Oligonukleotid besteht, ausgewählt aus der Gruppe bestehend aus:
einem fluoreszenzmarkierten Oligonukleotid mit der Basensequenz gezeigt in SEQ ID NR: 5, wobei die 3' Endbase von der Basensequenz mit einem Fluoreszenzfarbstoff markiert ist;
einem fluoreszenzmarkierten Oligonukleotid mit der Basensequenz gezeigt in SEQ ID NR: 15, wobei die 3' Endbase von der Basensequenz mit einem Fluoreszenzfarbstoff markiert ist; und
einem fluoreszenzmarkierten Oligonukleotid mit der Basensequenz gezeigt in SEQ ID NR: 16, wobei die 3' Endbase von der Basensequenz mit einem Fluoreszenzfarbstoff markiert ist.

2. Die Sonde zum Erkennen eines Polymorphismus gemäß Anspruch 1, **dadurch gekennzeichnet, dass** das fluoreszenzmarkierte Oligonukleotid Fluoreszenz emittiert, wenn das Oligonukleotid nicht mit einer Zielsequenz hybridisiert, und dass die Fluoreszenzintensität von dem Oligonukleotid abnimmt oder ansteigt, wenn das Oligonukleotid mit der Zielsequenz hybridisiert.

3. Die Sonde zum Erkennen eines Polymorphismus gemäß Anspruch 2, **dadurch gekennzeichnet, dass** das fluoreszenzmarkierte Oligonukleotid Fluoreszenz emittiert, wenn das Oligonukleotid nicht mit einer Zielsequenz hybridisiert, und dass die Fluoreszenzintensität von dem Oligonukleotid abnimmt, wenn das Oligonukleotid mit der Zielsequenz hybridisiert.

4. Die Sonde zum Erkennen eines Polymorphismus gemäß irgendeinem von den Ansprüchen 1 bis 3, **dadurch gekennzeichnet, dass** die Sonde eine Sonde für Schmelzkurvenanalyse ist.

5. Ein Verfahren zum Erkennen eines Polymorphismus von dem HGF Gen, **dadurch gekennzeichnet, dass** die Sonde gemäß irgendeinem von den Ansprüchen 1 bis 4 verwendet wird.

6. Das Verfahren zum Erkennen eines Polymorphismus von dem HGF Gen gemäß Anspruch 5, das Verfahren **gekennzeichnet durch** aufweisend:
(I) einen Schritt von Kombinieren der Sonde zum Erkennen eines Polymorphismus gemäß irgendeinem von den Ansprüchen 1 bis 4 mit einer einzelsträngigen Nukleinsäure in einer Probe, um ein Hybrid zwischen dem fluoreszenzmarkierten Oligonukleotid und der einzelsträngigen Nukleinsäure zu erhalten;
(II) einen Schritt von Ändern der Temperatur von der Probe beinhaltend das Hybrid, um das Hybrid zu dissoziieren, gefolgt von Messen von Änderungen in dem Fluoreszenzsignal aufgrund der Dissoziation von dem Hybrid;
(III) einen Schritt von Evaluieren von Tm, welches die Dissoziationstemperatur von dem Hybrid basierend auf der Änderung in dem Fluoreszenzsignal ist;
(IV) einen Schritt von Erkennen der Anwesenheit von dem Polymorphismus von dem HGF Gen basierend auf der Tm.

7. Das Verfahren zum Erkennen eines Polymorphismus von dem HGF Gen gemäß Anspruch 6, das Verfahren **gekennzeichnet durch** aufweisend einen Schritt von Amplifizieren der Nukleinsäure vor dem Erhalten des Hybrids in (I) oder gleichzeitig mit dem Erhalten des Hybrids in (I).

8. Ein Kit zum Erkennen eines Polymorphismus von dem HGF Gen, das Kit **gekennzeichnet durch** aufweisend die Sonde zum Erkennen eines Polymorphismus gemäß irgendeinem von den Ansprüchen 1 bis 4.

9. Das Kit zum Erkennen eines Polymorphismus von dem HGF Gen gemäß Anspruch 8, das Kit **gekennzeichnet durch** aufweisend einen Primer imstande zum Amplifizierten, als ein Template, einer Region aufweisend eine Sequenz, die hybridisiert mit zumindest einem fluoreszenzmarkierten Oligonukleotid ausgewählt aus der Gruppe bestehend aus einem fluoreszenzmarkierten Oligonukleotid mit der Basensequenz gezeigt in SEQ ID NR: 5, wobei die 3' Endbase von der Basensequenz mit einem Fluoreszenzfarbstoff markiert ist, einem fluoreszenzmarkierten Oligonukleotid mit der Basensequenz gezeigt in SEQ ID NR: 15, wobei die 3' Endbase von der Basensequenz mit einem Fluoreszenzfarbstoff markiert ist, und einem fluoreszenzmarkierten Oligonukleotid mit der Basensequenz gezeigt in SEQ ID NR: 16, wobei die 3' Endbase von der Basensequenz mit einem Fluoreszenzfarbstoff markiert ist.

## Revendications

1. Une sonde pour détecter un polymorphisme du gène HGF, **caractérisée en ce que** la sonde consiste en un oligonucléotide marqué par fluorescence choisi dans le groupe constitué de :
un oligonucléotide marqué par fluorescence ayant la séquence de bases montrée à la SEQ ID NO:5, dans lequel la base de l'extrémité 3' de la séquence de bases est marquée avec un colorant fluorescent ;
un oligonucléotide marqué par fluorescence ayant la séquence de bases montrée à la SEQ ID NO:15, dans lequel la base de l'extrémité 3' de la séquence de bases est marquée avec un colorant fluorescent ;
un oligonucléotide marqué par fluorescence ayant la séquence de bases montrée à la SEQ ID NO:16, dans lequel la base de l'extrémité 3' de la séquence de bases est marquée avec un colorant fluorescent.

2. La sonde pour détecter un polymorphisme selon la revendication 1, **caractérisée en ce que** l'oligonucléotide marqué par fluorescence émet de la fluorescence quand l'oligonucléotide ne s'hybride pas avec une séquence cible et que l'intensité de fluorescence de l'oligonucléotide diminue ou augmente quand l'oligonucléotide s'hybride à la séquence cible.

3. La sonde pour détecter un polymorphisme selon la revendication 2, **caractérisée en ce que** l'oligonucléotide marqué par fluorescence émet de la fluorescence quand l'oligonucléotide ne s'hybride pas avec une séquence cible et que l'intensité de fluorescence de l'oligonucléotide diminue quand l'oligonucléotide s'hybride à la séquence cible.

4. La sonde pour détecter un polymorphisme selon une quelconque des revendications 1 à 3, **caractérisée en ce que** la sonde est une sonde pour analyse de courbe de fusion.

5. Une méthode pour détecter un polymorphisme du gène HGF, **caractérisée en ce que** la sonde selon une quelconque des revendications 1 à 4 est utilisée.

6. La méthode pour détecter un polymorphisme du gène HGF selon la revendication 5, la méthode caractérisée en comprenant :
(I) une étape de combiner dans un échantillon la sonde pour détecter un polymorphisme selon une quelconque des revendications 1 à 4 avec un acide nucléique simple brin pour obtenir un hybride entre l'oligonucléotide marqué par fluorescence et l'acide nucléique simple brin ;
(II) une étape de varier la température de l'échantillon incluant l'hybride à dissocier pour dissocier l'hybride, suivie de la mesure des changements de signal fluorescent dus à la dissociation de l'hybride ;
(III) une étape d'évaluation de Tm qui est la température de dissociation de l'hybride basée sur un changement au signal fluorescent ;
(IV) une étape de détection de la présence du polymorphisme du gène HGF basée sur la Tm.

7. La méthode pour détecter un polymorphisme du gène HGF selon la revendication 6, la méthode caractérisée en comprenant une étape d'amplification de l'acide nucléique avant d'obtenir l'hybride en (I) ou simultanément avec l'obtention de l'hybride en (I).

8. Un kit pour détecter un polymorphisme du gène HGF, le kit caractérisé en comprenant la sonde pour détecter un polymorphisme selon une quelconque des revendications 1 à 4.

9. Le kit pour détecter un polymorphisme du gène HGF selon la revendication 8, le kit caractérisé en comprenant une amorce capable d'amplifier comme région matrice comprenant une séquence qui s'hybride avec au moins un oligonucléotide marqué par fluorescence choisi dans le groupe constitué d'un oligonucléotide marqué par fluorescence ayant la séquence de bases montrée à la SEQ ID NO:5 dans lequel la base de l'extrémité 3' de la séquence de bases est marquée avec un colorant fluorescent, un oligonucléotide marqué par fluorescence ayant la séquence de bases montrée à la SEQ ID NO:15 dans lequel la base de l'extrémité 3' de la séquence de bases est marquée avec un colorant fluorescent et un oligonucléotide marqué par fluorescence ayant la séquence de bases montrée à la SEQ ID NO:16 dans lequel la base de l'extrémité 3' de la séquence de bases est marquée avec un colorant fluorescent.


### SEQUENCE LISTING

<110> Arkray, Inc.
<120> Probe for detecting poly A repeat number polymorphism of HGF gene and
   uses thereof
<130> 42.13.112988
<150> JP 2011-104503
   <151> 2011-05-09
<150> JP 2012-089507
   <151> 2012-04-10
<160> 79
<170> PatentIn version 3.1
<210> 1
   <211> 49
   <212> DNA
   <213> An artificial sequence
<400> 1
   gccagcgttc aatctgagcc atgatcaaac tcttcaaaaa aaaaaaaaa 49
<210> 2
   <211> 32
   <212> DNA
   <213> An artificial sequence
<400> 2
   aaaaaaaaaa aaaagctgcc tcccgtagga gt 32
<210> 3
   <211> 249
   <212> DNA
   <213> Homo sapiens
<400> 3
   gggtgtggta ttgtggggcc aaaataagca agcaaaacaa aagcacgcag attgtcagat      60
   gaaaaaattg cccctgcccc atgggctcag agcaggcagc tttttttttt tttttttttt     120
   tttttttttt tcacaaactg ccaaactcac gaaataccac acatttaccc aaagtataca     180
   aaataaactt gtctgatctg actttttaag agaaagtcca agaaacagtc attgtccata     240
   gcctgtccc                                                             249
<210> 4
   <211> 46
   <212> DNA
   <213> An artificial sequence
<400> 4
   cagtttgtga aaaaaaaaaa aaaaaaaaaa aaaaaaaaaa gctgcc 46
<210> 5
   <211> 42
   <212> DNA
   <213> An artificial sequence
<400> 5
   ttgtgaaaaa aaaaaaaaaa aaaaaaaaaa aaaaaagctg cc 42
<210> 6
   <211> 12
   <212> DNA
   <213> An artificial sequence
<400> 6
   ttgtgagctg cc 12
<210> 7
   <211> 46
   <212> DNA
   <213> An artificial sequence
<400> 7
   tttgtgaaaa aaaaaaaaaa aaaaaaaaaa aaaaaaagct gcctgc 46
<210> 8
   <211> 42
   <212> DNA
   <213> An artificial sequence
<400> 8
   tgaaaaaaaa aaaaaaaaaa aaaaaaaaaa aaagctgcct gc 42
<210> 9
   <211> 11
   <212> DNA
   <213> An artificial sequence
<400> 9
   gagctgcctg c 11
<210> 10
   <211> 46
   <212> DNA
   <213> An artificial sequence
<400> 10
   agagcaggca gctttttttt tttttttttt tttttttttt tttcac 46
<210> 11
   <211> 36
   <212> DNA
   <213> An artificial sequence
<400> 11
   gctttttttt tttttttttt tttttttttt tttcac 36
<210> 12
   <211> 8
   <212> DNA
   <213> An artificial sequence
<400> 12
   gctttcac 8
<210> 13
   <211> 60
   <212> DNA
   <213> An artificial sequence
<400> 13
   ctcagagcag gcagcttttt tttttttttt tttttttttt ttttttcaca aactgccaaa 60
<210> 14
   <211> 60
   <212> DNA
   <213> An artificial sequence
<400> 14
   gggctcagag caggcagctt tttttttttt tttttttttt ttttcacaaa ctgccaaact 60
<210> 15
   <211> 45
   <212> DNA
   <213> An artificial sequence
<400> 15
   ttgtgaaaaa aaaaaaaaaa aaaaaaaaaa aaaaaagctg cctgc 45
<210> 16
   <211> 36
   <212> DNA
   <213> An artificial sequence
<400> 16
   gctttttttt tttttttttt tttttttttt tttcac 36
<210> 17
   <211> 60
   <212> DNA
   <213> An artificial sequence
<400> 17
   tttggcagtt tgtgaaaaaa aaaaaaaaaa aaaaaaaaaa aaaaagctgc ctgctctgag 60
<210> 18
   <211> 60
   <212> DNA
   <213> An artificial sequence
<400> 18
   agtttggcag tttgtgaaaa aaaaaaaaaa aaaaaaaaaa aagctgcctg ctctgagccc 60
<210> 19
   <211> 47
   <212> DNA
   <213> An artificial sequence
<400> 19
   ttgtgaaaaa aaaaaaaaaa aaaaaaaaaa aaaaaagctg cctgctc 47
<210> 20
   <211> 32
   <212> DNA
   <213> An artificial sequence
<400> 20
   gggacaggct atggacaatg actgtttctt gg 32
<210> 21
   <211> 28
   <212> DNA
   <213> An artificial sequence
<400> 21
   gggtgtggta ttgtggggcc aaaataag 28
<210> 22
   <211> 248
   <212> DNA
   <213> An artificial sequence
<400> 22
   gggtgtggta ttgtggggcc aaaataagca agcaaaacaa aagcacgcag attgtcagat 60
   gaaaaaattg cccctgcccc atgggctcag agcaggcagc tttttttttt tttttttttt 120
   tttttttttt cacaaactgc caaactcacg aaataccaca catttaccca aagtatacaa 180
   aataaacttg tctgatctga ctttttaaga gaaagtccaa gaaacagtca ttgtccatag 240
   cctgtccc 248
<210> 23
   <211> 244
   <212> DNA
   <213> An artificial sequence
<400> 23
   gggtgtggta ttgtggggcc aaaataagca agcaaaacaa aagcacgcag attgtcagat 60
   gaaaaaattg cccctgcccc atgggctcag agcaggcagc tttttttttt tttttttttt 120
   ttttttcaca aactgccaaa ctcacgaaat accacacatt tacccaaagt atacaaaata 180
   aacttgtctg atctgacttt ttaagagaaa gtccaagaaa cagtcattgt ccatagcctg 240
   tccc 244
<210> 24
   <211> 239
   <212> DNA
   <213> An artificial sequence
<400> 24
   gggtgtggta ttgtggggcc aaaataagca agcaaaacaa aagcacgcag attgtcagat     60
   gaaaaaattg cccctgcccc atgggctcag agcaggcagc tttttttttt tttttttttt    120
   tcacaaactg ccaaactcac gaaataccac acatttaccc aaagtataca aaataaactt    180
   gtctgatctg actttttaag agaaagtcca agaaacagtc attgtccata gcctgtccc     239
<210> 25
   <211> 36
   <212> DNA
   <213> An artificial sequence
<400> 25
   aaaaaaaaaa aaaaaaaaaa aaaaaaaaaa gctgcc 36
<210> 26
   <211> 34
   <212> DNA
   <213> An artificial sequence
<400> 26
   aaaaaaaaaa aaaaaaaaaa aaaaaaaagc tgcc 34
<210> 27
   <211> 32
   <212> DNA
   <213> An artificial sequence
<400> 27
   aaaaaaaaaa aaaaaaaaaa aaaaaagctg cc 32
<210> 28
   <211> 39
   <212> DNA
   <213> An artificial sequence
<400> 28
   gcaggcagct tttttttttt tttttttttt ttttttttt 39
<210> 29
   <211> 37
   <212> DNA
   <213> An artificial sequence
<400> 29
   gcaggcagct tttttttttt tttttttttt ttttttt 37
<210> 30
   <211> 35
   <212> DNA
   <213> An artificial sequence
<400> 30
   gcaggcagct tttttttttt tttttttttt ttttt 35
<210> 31
   <211> 33
   <212> DNA
   <213> An artificial sequence
<400> 31
   tttttttttt tttttttttt tttttttttt cac 33
<210> 32
   <211> 31
   <212> DNA
   <213> An artificial sequence
<400> 32
   tttttttttt tttttttttt ttttttttca c 31
<210> 33
   <211> 29
   <212> DNA
   <213> An artificial sequence
<400> 33
   tttttttttt tttttttttt ttttttcac 29
<210> 34
   <211> 7
   <212> DNA
   <213> An artificial sequence
<400> 34
   agctgcc 7
<210> 35
   <211> 8
   <212> DNA
   <213> An artificial sequence
<400> 35
   gagctgcc 8
<210> 36
   <211> 12
   <212> DNA
   <213> An artificial sequence
<400> 36
   ttgtgagctg cc 12
<210> 37
   <211> 9
   <212> DNA
   <213> An artificial sequence
<400> 37
   aaagctgcc 9
<210> 38
   <211> 12
   <212> DNA
   <213> An artificial sequence
<400> 38
   aaaaaagctg cc 12
<210> 39
   <211> 37
   <212> DNA
   <213> An artificial sequence
<400> 39
   aaaaaaaaaa aaaaaaaaaa aaaaaaaaaa agctgcc 37
<210> 40
   <211> 42
   <212> DNA
   <213> An artificial sequence
<400> 40
   ttgtgaaaaa aaaaaaaaaa aaaaaaaaaa aaaaaagctg cc 42
<210> 41
   <211> 44
   <212> DNA
   <213> An artificial sequence
<400> 41
   aaaaaaaaaa aaaaaaaaaa aaaaaaaaaa agctgcctgc tctg 44
<210> 42
   <211> 46
   <212> DNA
   <213> An artificial sequence
<400> 42
   cagtttgtga aaaaaaaaaa aaaaaaaaaa aaaaaaaaaa gctgcc 46
<210> 43
   <211> 50
   <212> DNA
   <213> An artificial sequence
<400> 43
   aaaaaaaaaa aaaaaaaaaa aaaaaaaaaa agctgcctgc tctgagccca 50
<210> 44
   <211> 52
   <212> DNA
   <213> An artificial sequence
<400> 44
   aaaaaaaaaa aaaaaaaaaa aaaaaaaaaa agctgcctgc tctgagccca tg 52
<210> 45
   <211> 55
   <212> DNA
   <213> An artificial sequence
<400> 45
   aaaaaaaaaa aaaaaaaaaa aaaaaaaaaa agctgcctgc tctgagccca tgggg 55
<210> 46
   <211> 56
   <212> DNA
   <213> An artificial sequence
<400> 46
   aaaaaaaaaa aaaaaaaaaa aaaaaaaaaa agctgcctgc tctgagccca tggggc 56
<210> 47
   <211> 57
   <212> DNA
   <213> An artificial sequence
<400> 47
   aaaaaaaaaa aaaaaaaaaa aaaaaaaaaa agctgcctgc tctgagccca tggggca 57
<210> 48
   <211> 94
   <212> DNA
   <213> An artificial sequence
<400> 48
   gaaaaaaaaa aaaaaaaaaa aaaaaaaaaa aagctgcctg ctctgagccc atggggcagg 60
   ggcaattttt tcatctgaca atctgcgtgc tttt 94
<210> 49
   <211> 99
   <212> DNA
   <213> An artificial sequence
<400> 49
   gaaaaaaaaa aaaaaaaaaa aaaaaaaaaa aagctgcctg ctctgagccc atggggcagg 60
   ggcaattttt tcatctgaca atctgcgtgc ttttgtttt 99
<210> 50
   <211> 10
   <212> DNA
   <213> An artificial sequence
<400> 50
   agctgcctgc 10
<210> 51
   <211> 11
   <212> DNA
   <213> An artificial sequence
<400> 51
   gagctgcctg c 11
<210> 52
   <211> 12
   <212> DNA
   <213> An artificial sequence
<400> 52
   aaagctgcct gc 12
<210> 53
   <211> 15
   <212> DNA
   <213> An artificial sequence
<400> 53
   aaaaaagctg cctgc 15
<210> 54
   <211> 37
   <212> DNA
   <213> An artificial sequence
<400> 54
   aaaaaaaaaa aaaaaaaaaa aaaaaaaaaa agctgcc 37
<210> 55
   <211> 40
   <212> DNA
   <213> An artificial sequence
<400> 55
   aaaaaaaaaa aaaaaaaaaa aaaaaaaaaa agctgcctgc 40
<210> 56
   <211> 42
   <212> DNA
   <213> An artificial sequence
<400> 56
   tgaaaaaaaa aaaaaaaaaa aaaaaaaaaa aaagctgcct gc 42
<210> 57
   <211> 42
   <212> DNA
   <213> An artificial sequence
<400> 57
   aaaaaaaaaa aaaaaaaaaa aaaaaaaaaa agctgcctgc tc 42
<210> 58
   <211> 46
   <212> DNA
   <213> An artificial sequence
<400> 58
   tttgtgaaaa aaaaaaaaaa aaaaaaaaaa aaaaaaagct gcctgc 46
<210> 59
   <211> 55
   <212> DNA
   <213> An artificial sequence
<400> 59
   aaaaaaaaaa aaaaaaaaaa aaaaaaaaaa agctgcctgc tctgagccca tgggg 55
<210> 60
   <211> 57
   <212> DNA
   <213> An artificial sequence
<400> 60
   aaaaaaaaaa aaaaaaaaaa aaaaaaaaaa agctgcctgc tctgagccca tggggca 57
<210> 61
   <211> 94
   <212> DNA
   <213> An artificial sequence
<400> 61
   gaaaaaaaaa aaaaaaaaaa aaaaaaaaaa aagctgcctg ctctgagccc atggggcagg 60
   ggcaattttt tcatctgaca atctgcgtgc tttt 94
<210> 62
   <211> 99
   <212> DNA
   <213> An artificial sequence
<400> 62
   gaaaaaaaaa aaaaaaaaaa aaaaaaaaaa aagctgcctg ctctgagccc atggggcagg 60
   ggcaattttt tcatctgaca atctgcgtgc ttttgtttt 99
<210> 63
   <211> 5
   <212> DNA
   <213> An artificial sequence
<400> 63
   ctcac 5
<210> 64
   <211> 6
   <212> DNA
   <213> An artificial sequence
<400> 64
   cttcac 6
<210> 65
   <211> 6
   <212> DNA
   <213> An artificial sequence
<400> 65
   tttcac 6
<210> 66
   <211> 7
   <212> DNA
   <213> An artificial sequence
<400> 66
   ctttcac 7
<210> 67
   <211> 7
   <212> DNA
   <213> An artificial sequence
<400> 67
   ttttcac 7
<210> 68
   <211> 8
   <212> DNA
   <213> An artificial sequence
<400> 68
   cttttcac 8
<210> 69
   <211> 8
   <212> DNA
   <213> An artificial sequence
<400> 69
   tttttcac 8
<210> 70
   <211> 10
   <212> DNA
   <213> An artificial sequence
<400> 70
   tttttttcac 10
<210> 71
   <211> 34
   <212> DNA
   <213> An artificial sequence
<400> 71
   tttttttttt tttttttttt tttttttttt tcac 34
<210> 72
   <211> 35
   <212> DNA
   <213> An artificial sequence
<400> 72
   cttttttttt tttttttttt tttttttttt ttcac 35
<210> 73
   <211> 36
   <212> DNA
   <213> An artificial sequence
<400> 73
   gctttttttt tttttttttt tttttttttt tttcac 36
<210> 74
   <211> 46
   <212> DNA
   <213> An artificial sequence
<400> 74
   agagcaggca gctttttttt tttttttttt tttttttttt tttcac 46
<210> 75
   <211> 120
   <212> DNA
   <213> An artificial sequence
<400> 75
   cttttttttt tttttttttt tttttttttt ttcacaaact gccaaactca cgaaatacca 60
   cacatttacc caaagtatac aaaataaact tgtctgatct gactttttaa gagaaagtcc 120
<210> 76
   <211> 145
   <212> DNA
   <213> An artificial sequence
<400> 76
   cttttttttt tttttttttt tttttttttt ttcacaaact gccaaactca cgaaatacca 60
   cacatttacc caaagtatac aaaataaact tgtctgatct gactttttaa gagaaagtcc 120
   aagaaacagt cattgtccat agcct 145
<210> 77
   <211> 56
   <212> DNA
   <213> An artificial sequence
<400> 77
   tttttttttt tttttttttt tttttttttt tcacaaactg ccaaactcac gaaata 56
<210> 78
   <211> 60
   <212> DNA
   <213> An artificial sequence
<400> 78
   tttttttttt tttttttttt tttttttttt tcacaaactg ccaaactcac gaaataccac 60
<210> 79
   <211> 80
   <212> DNA
   <213> An artificial sequence
<400> 79
   tttttttttt tttttttttt tttttttttt tcacaaactg ccaaactcac gaaataccac     60
   acatttaccc aaagtataca                                                 80